# EUROPÄISCHE PATENTSCHRIFT

(11) **EP 4 442 190 B1**
(45) Veröffentlichungstag und Bekanntmachung des Hinweises auf die Patenterteilung: **08.07.2026**
(21) Anmeldenummer: 24168188.1
(22) Anmeldetag: 03.04.2024
(51) Int. Cl.: A61B 3/00, G01B 9/02055, A61B 3/12, A61B 3/10, G01B 9/02091

(54) **OCT-SYSTEM UND VERFAHREN ZUM BETRIEB DESSELBEN**
OCT SYSTEM AND METHOD OF OPERATING THE SAME
SYSTÈME OCT ET SON PROCÉDÉ DE FONCTIONNEMENT

(30) Priorität: 03.04.2023 DE 102023108489
(43) Veröffentlichungstag der Anmeldung: 09.10.2024
(73) Patentinhaber: Carl Zeiss Meditec AG, 07745 Jena (DE)
(72) Erfinder: Matz, Holger, 73447 Oberkochen (DE); Sorg, Benjamin, 73447 Oberkochen (DE)
(74) Vertreter: Gulde & Partner

(56) Entgegenhaltungen:
- DE-A1- 102008 041 284
- DE-A1- 102014 010 350
- US-A1- 2015 085 294

## Beschreibung

### Gegenstand der Erfindung

Die vorliegende Erfindung betrifft ein OCT-System und ein Verfahren zum Betrieb eines OCT-Systems, insbesondere zum Ermitteln eines im Strahlengang des OCT-Systems befindlichen optischen Instruments.

### Technologischer Hintergrund

Der Einsatz von technologischen Hilfsmitteln ist fester Bestandteil der modernen Medizin. Verschiedenste bildgebende Verfahren werden mittlerweile in der Chirurgie ebenso selbstverständlich eingesetzt, wie in der Diagnostik. Das Verwenden bildgebender Verfahren ermöglicht dabei die Darstellung sowie Diskriminierung vielfältiger Strukturen im Patienten und die vom Patienten gewonnenen Bilddaten können somit vorteilhaft nicht nur in der Diagnostik, sondern auch in therapeutischen und chirurgischen Verfahren eingesetzt werden.

Operationsmikroskope finden unter anderem regelmäßige Anwendung bei ophthalmologischen Eingriffen, beispielsweise in der Kataraktchirurgie und der Retinachirurgie. Mittels des Operationsmikroskops erfolgt dabei während des ophthalmologischen Eingriffs eine kontinuierliche oder semi-kontinuierliche Abbildung von Strukturen des Auges. So ist in der Retinachirurgie regelmäßig eine Abbildung des Augenhintergrundes (Fundus) notwendig. Je nach chirurgischem Eingriff können aber auch Abbildungen mittlerer Augenabschnitte oder der Netzhaut(-peripherie) notwendig sein.

Neben der Erfassung der Oberfläche eines Sichtfelds mittels Operationsmikroskop existieren mittlerweile auch Methoden zum Erfassen von Tiefeninformationen des Sichtfelds. Diese Methoden umfassen die optische Kohärenztomographie *(optical coherence tomography, OCT*), welche die dreidimensionale mikroskopische Abbildung von optisch transparenten und/oder reflektierenden Objekten und somit die Aufnahme von Volumenbildern des biologischen Gewebes im betrachteten Sichtfeld erlaubt. Bei der optischen Kohärenztomographie (OCT) handelt es sich im Wesentlichen um eine interferometrische Methode unter Verwendung von breitbandigem Licht mit geringer Kohärenzlänge. Systeme zum Erfassen von OCT Daten weisen daher in der Regel ein Interferometer und eine breitbandige Lichtquelle mit spektraler Breite von mehr als 1% der zentralen Wellenlänge auf.

Die Erfassung von OCT Daten kann sequentiell oder parallel erfolgen. Die sequentielle **Erfassung** von OCT Daten erfolgt beispielsweise indem ein kohärenzarmer Quelllichtstrahl an einem Strahlteiler in einen Probenstrahl und in einen Referenzstrahl geteilt wird, die durch zwei Arme eines Interferometers geschickt werden, wobei im Referenzstrahlengang ein beweglicher Referenzspiegel und im Objektstrahlengang das zu untersuchende Objekt angeordnet sind. Durch Verschiebung des Referenzspiegels kann ein Gangunterschied zwischen Objekt- und Referenzstrahl und somit die vermessene Tiefe eingestellt werden. Mittels eines Spiegels im Objektstrahlengang wird der Objektstrahl zweidimensional über die Probe gerastert, was im Ergebnis eine dreidimensionale Abtastung der Probe ermöglicht.

Bei solch einer Erfassung von OCT Daten in der Zeitdomäne *(time domain OCT - TD OCT)* korrespondiert die spektrale Breite der Lichtquelle Δλ zu einer Kohärenzlänge L_{C} von L_{C}=λ*/Δλ. Die axiale Auflösung eines OCT-Systems korrespondiert zur Kohärenzlänge L_{C} des eingesetzten Lichts und bezeichnet das Auflösungsvermögen von Objekten, die entlang der optischen Achse einen Abstand von zumindest der Kohärenzlänge aufweisen. Beispielsweise hat eine Lichtquelle im Nahinfrarotbereich mit zentraler Wellenlänge von 800 nm und einer spektralen Breite von 80 nm eine Kohärenzlänge von 7 µm und ein OCT System mit einer solchen Quelle hat somit eine axiale Auflösung von etwa 1-10 µm. Die transversale Auflösung eines OCT Systems ist durch die im Objektstrahlengang verwendete Optik bestimmt, insbesondere durch die das Licht auf das zu untersuchende Objekt fokussierende Objektlinse.

Eine sequentielle Erfassung von OCT Daten ist auch in der Frequenzdomäne möglich *(frequency domain OCT - FD* OCT), wobei in der Regel zwischen der Verwendung einer durchstimmbaren Quelle *(swept source* OCT) und der Verwendung eines dispersiven Detektors *(spectral domain OCT-* SD OCT) unterschieden wird. Bei der *swept source OCT* wird die Frequenz der Anregungslichtquelle, beispielsweise einem Laser, durchgestimmt, wodurch ein Gangunterschied zwischen Proben- und Referenzstrahl und somit die abgetastete Probentiefe auch ohne verschiebbaren Referenzspiegel variiert werden kann. Bei der SD OCT wird ebenfalls eine breitbandige Lichtquelle verwendet, jedoch werden die Frequenzkomponenten des Interferenzsignals vor der Detektion separiert, beispielsweise durch ein optisches Gitter.

Mittels OCT sind Schnitt- und Volumendaten von biologischem Gewebe erfassbar und kann der Informationsgehalt für einen Operateur deutlich erhöht werden. Somit ist eine Integration von OCT in Operationsmikroskope sinnvoll, um sowohl Videodaten der Oberfläche eines gewünschten Sichtfelds als auch Tiefen- und/oder Schnittbilder des Sichtfelds darzustellen. Auch bei ophthalmologischen Eingriffen bietet die Anwendung von OCT viele Vorteile, indem Schnittdaten in der Diagnostik beispielsweise detailliert Auskunft über Hornhautkrümmungen und Ablösungen von Netzhautkomponenten geben können und bei chirurgischen Eingriffen beispielsweise über die Relativlage von chirurgischem Werkzeug und Augenstrukturen.

Das Abbilden von Strukturen des Auges optisch mittels Operationsmikroskop oder mittels OCT-Visualisierung erfordert regelmäßig das Einbringen zusätzlicher optischer Elemente in den Strahlengang. Beispiele hierfür sind das Einbringen von Funduslupen in den Strahlengang zur Abbildung des Augenhintergrundes sowie das Aufsetzen von Kontaktgläsern zur Abbildung diverser Augenabschnitte. Funduslupen kommen beispielsweise im Zeiss Resight 700 und im Zeiss Opmi Lumera 700 mit Rescan 700 zur Anwendung. Diese zusätzlichen optischen Elemente sind dabei in der Regel im Sterilbereich operativer Eingriffe angeordnet und/oder werden direkt mit dem zu untersuchenden Auge in Kontakt gebracht. Eine Integration dieser optischen Elemente in ein Operationsmikroskop oder ein OCT-System selbst ist daher nur schwer zu realisieren.

Gleichzeitig ist aber die genaue Kenntnis aller im Strahlengang des Operationsmikroskops oder OCT-Systems befindlicher optischen Elemente unerlässlich, insbesondere aber nicht nur für eine quantitative Auswertung von mit dem Operationsmikroskop erfassten Bildinformationen. Generell kann die fehlende oder falsche Berücksichtigung solcher zusätzlichen optischen Elemente im Strahlengang zu Skalierungsfehlern bei der Auswertung und/oder Darstellung der erfassten Bildsignale oder OCT-Signale führen. Dies kann die medizintechnische Navigation als auch die Diagnostik negativ beeinflussen, beispielsweise da anormale Strukturgrößen häufig als Indikator für krankhafte Veränderungen genutzt werden.

Bisherige Lösungen von zusätzlich im Strahlengang angeordneten optischen Instrumenten basieren in der Regel auf Nutzereingaben zur Auswahl der im Strahlengang befindlichen optischen Instrumente. Eingabefehler sind dabei nur schwer zu vermeiden. Die DE 102014010350 A1 offenbart ein Augenchirurgiesystem, das zum überlagerten Darstellen eines präoperativen und eines intraoperativen OCT-Bilds ausgebildet ist. Die US 20150085294 A1 offenbart ein ophthalmologisches OCT-System, bei dem die Komponenten des optischen Strangs identifiziert und deren Positionen ermittelt werden können, beispielsweise um vor einer Untersuchung sicherzustellen, dass die korrekten optischen Komponenten installiert wurden.

Die Aufgabe der vorliegenden Erfindung ist es daher, ein verbessertes Verfahren und ein verbessertes System zum Berücksichtigen zusätzlicher optischer Elemente im Strahlengang eines OCT-Systems bereitzustellen, welches die Nachteile des Standes der Technik überwindet oder verringert, insbesondere die Gefahr von Bedien- oder Eingabefehlern.

### Beschreibung der Erfindung

Die erfindungsgemäße Aufgabe wird gelöst durch die Gegenstände der unabhängigen Patentansprüche. Bevorzugte Weiterbildungen sind Gegenstand der Unteransprüche.

Ein erster Aspekt der vorliegenden Offenbarung betrifft ein Verfahren zum Betrieb eines OCT-Systems, bevorzugt eines in ein medizintechnisches Gerät integrierten OCT-Systems. Bei dem medizintechnischen Gerät handelt es sich beispielsweise um ein Operationsmikroskop. Das medizintechnische Gerät ist dabei bevorzugt zur multimodalen Bilderfassung und Bilddarstellung ausgebildet, insbesondere zur Erfassung von OCT-Signalen und Bildsignalen. In einem ersten Schritt des Verfahrens gemäß der vorliegenden Offenbarung erfolgt ein Erfassen eines OCT-Signals eines in einem Strahlengang des OCT-Systems eingebrachten optischen Instruments. Alternativ oder zusätzlich erfolgt das Erfassen eines OCT-Signals einer in den Strahlengang eingebrachten anatomischen Struktur. Das OCT-Signal wird dabei bevorzugt erfasst, indem ein Lichtsignal erzeugt und mittels eines Interferometers des OCT-Systems teilweise als Probenstrahl auf das optische Instrument und/oder die vorbestimmte Struktur gelenkt und anschließend in dem Interferometer mit einem ebenfalls aus dem Lichtsignal erzeugten Referenzstrahl zur Überlagerung gebracht wird, um ein Interferenzmuster zu erzeugen. Das optische Instrument und/oder die anatomische Struktur ist dabei in einem Strahlengang des OCT-Systems angeordnet, wobei es sich ferner bevorzugt gleichzeitig auch um den Strahlengang des medizintechnischen Geräts, wie ein Operationsmikroskop, handelt.

Bei dem OCT-Signal handelt es sich bevorzugt um ein Interferenzsignal, wobei die Modulation der Einhüllenden des Interferenzsignals Reflektions- beziehungsweise Streueigenschaften des optischen Instruments und/oder der vorbestimmten Struktur kodiert. Mittels eines Scanmechanismus kann der Strahlengang beziehungsweise das darin befindliche optische Instrument und/oder die darin befindliche vorbestimmte Struktur zweidimensional in einer über den Gangunterschied von Referenz- und Probenstrahl eingestellten Tiefe abgerastert werden. Das OCT-Signal kodiert mithin Informationen zum Strahlengang beziehungsweise dem darin befindlichen optischen Instrument und/oder der darin befindlichen anatomischen Struktur, anhand derer rechnerisch ein zeitaufgelöstes OCT-Bild, beispielsweise mittels Volumen-Rendering, Ray-Tracing und/oder Ray-Marching erstellbar ist. Dem Fachmann sind Verfahren zum Erzeugen von OCT-Bildern aus OCT-Signalen bekannt. Bei dem OCT-Signal handelt es sich bevorzugt um einen A-Scan, also um ein an einem einzelnen Scanpunkt entlang der Tiefenrichtung (der optischen Achse) gerastertem Signal. Der A-Scan zeigt mithin das Reflektionsprofil des optischen Instruments und/oder der vorbestimmten Struktur in Tiefenrichtung. Bei dem OCT-Signal kann es sich jedoch bevorzugt auch um einen B-Scan oder einen C-Scan handeln.

In einem Verfahren gemäß einem Beispiel erfolgt in einem weiteren Schritt das Ermitteln einer Eigenschaft des optischen Instruments anhand des OCT-Signals. Das Ermitteln der Eigenschaft kann dabei mit oder ohne Erzeugung eines OCT-Bilds erfolgen. Die Eigenschaft wird also bevorzugt unmittelbar anhand des OCT-Signals, sprich anhand der Rohdaten, ermittelt. Alternativ wird anhand des OCT-Signals zunächst ein OCT-Bild ermittelt und wird die Eigenschaft des optischen Instruments anhand des OCT-Bilds ermittelt. Bei der Eigenschaft des optischen Instruments handelt es sich bevorzugt um eine optische Eigenschaft des optischen Instruments und/oder um eine geometrische Eigenschaft des optischen Instruments. Eine optische Eigenschaft des optischen Instruments ergibt sich bevorzugt aus der geometrischen Eigenschaft (Form) des optischen Instruments. Alternativ hängt eine optische Eigenschaft des optischen Instruments von einer Oberflächenbeschaffenheit und/oder einem Herstellungsprozess des optischen Instruments ab. Somit kann anhand der ermittelten optischen Eigenschaft des optischen Instruments vorteilhaft auf eine geometrische Eigenschaft (Form), eine Oberflächenbeschaffenheit und/oder einen Herstellungsprozess des optischen Instruments und davon auf eine Kenngröße desselben geschlossen werden. Handelt es sich bei der Eigenschaft um eine geometrische Eigenschaft, wird diese ferner bevorzugt anhand des OCT-Bildes durch Algorithmen zur Bilderkennung ermittelt. Zur Bilderkennung können dem Fachmann bekannte Algorithmen zur automatischen Bilderkennung verwendet werden, beispielsweise auf Algorithmen des maschinellen Lernens basierende Algorithmen. Die Algorithmen umfassen beispielsweise Algorithmen zur Kantendetektion wie Canny, Sobel und Roberts, die verwendet werden können, um Grenzen zwischen verschiedenen Regionen im Bild zu erkennen und/oder Algorithmen zur Formanalyse, wie Fourier-Deskriptoren und Hu-Momente, um eine oder mehrere geometrische Eigenschaften, wie beispielsweise Form, Größe, Ausdehnung, Ausrichtung, Krümmung und/oder Symmetrie des optischen Instruments zu quantifizieren. Erfindungsgemäß erfolgt im Verfahren gemäß der vorliegenden Offenbarung das Ermitteln einer Position des optischen Instruments, insbesondere einer Position des optischen Instruments im Strahlengang des OCT-Systems und bevorzugt des Operationsmikrokops. Die Position des optischen Instruments beschreibt dabei die Raumlage des optischen Instruments in einem Koordinatensystem des OCT-Systems und bevorzugt des Operationsmikrokops, also beispielsweise als Relativlage zu anderen Komponenten des OCT-Systems und bevorzugt des Operationsmikroskops. Die Position des optischen Instruments kann ebenso eine Ausrichtung des optischen Instruments betreffen und diesbezüglich einen zu der geometrischen Form korrespondierenden Informationsgehalt aufweisen.

Erfindungsgemäß erfolgt alternativ oder zusätzlich das Ermitteln einer Eigenschaft der anatomischen Struktur anhand des OCT-Signals durch ein in den Strahlengang des OCT-Systems eingebrachtes optisches Instrument. Das Ermitteln erfolgt dabei bevorzugt computerimplementiert und das Ermitteln der Eigenschaft kann dabei mit oder ohne Erzeugung eines OCT-Bilds erfolgen. Die Eigenschaft wird also bevorzugt unmittelbar anhand des OCT-Signals, sprich anhand der Rohdaten, ermittelt. Alternativ wird anhand des OCT-Signals zunächst ein OCT-Bild ermittelt und wird die Eigenschaft der anatomischen Struktur anhand des OCT-Bilds ermittelt. Die Struktur ist dabei bevorzugt derart vorbestimmt, als das bei der Durchführung des Verfahrens gemäß der vorliegenden Offenbarung *a priori* Informationen zu der Struktur vorhanden sind. Mit anderen Worten wird keine beliebige Struktur, sondern ganz gezielt eine durch die a *priori* vorliegenden Informationen zumindest generisch bestimmte Struktur im Strahlengang ermittelt. Die Informationen betreffen mithin keine individuelle Struktur, sondern beispielsweise einen bestimmten Typ oder eine Art von Struktur, eine Klasse von Strukturen oder dergleichen. Bei der ermittelten Eigenschaft handelt es sich um eine Eigenschaft der individuellen in dem Strahlengang angeordneten Struktur. Die ermittelte Eigenschaft ist bevorzugt ebenfalls a priori anhand von Informationen generisch klassifiziert, beispielsweise indem vorbestimmt ist, welche Art von Eigenschaft der ermittelten Struktur bestimmt wird. Bei der Eigenschaft handelt es sich um eine Größe der vorbestimmten Struktur. Ebenso bevorzugt werden in diesem Schritt mehrere Eigenschaften der ermittelten Struktur oder Kombinationen von Eigenschaften ermittelt. Es handelt sich somit bevorzugt um scheinbare Eigenschaften der Struktur in dem OCT-Signal, nicht um die tatsächliche Eigenschaft der Struktur. So wird anhand des OCT-Signals eine scheinbare Größe der Struktur ermittelt, welche jedoch nicht der tatsächlichen Größe der Struktur entsprechen muss. In einem weiteren Schritt des erfindungsgemäßen Verfahrens erfolgt schließlich ein Ermitteln einer Kenngröße eines in den Strahlengang des OCT-Systems eingebrachten optischen Instruments anhand der Position des optischen Instruments. Mit anderen Worten werden Informationen zur (Relativ-)Lage des optischen Instruments verwendet, um auf eine Kenngröße insbesondere eine optische Kenngröße, und letztlich auf das optische Instrument selbst zu schließen. Somit werden die im Verfahren gemäß der vorliegenden Offenbarung ermittelten Informationen zu (Relativ-)Lage des optischen Instruments verwendet, um einen Rückschluss auf das in den Strahlengang des OCT-Systems und bevorzugt eines Operationsmikroskops eingebrachte optische Instrument zu erlangen. Alternativ oder zusätzlich und/oder wird eine Kenngröße des optischen Instruments anhand der ermittelten Eigenschaft der anatomischen Struktur bestimmt.

Bei dem optischen Instrument handelt es sich bevorzugt um eine Lupe, wie eine Ophthalmoskopierlupe, um ein Kontaktglas, um eine Linse, wie eine Vitrektomielinse, oder ähnliches. Bei der Kenngröße handelt es sich erfindungsgemäß um eine Eigenschaft des optischen Instruments, die eine Identifikation und bevorzugt eine Klassifizierung des optischen Instruments ermöglicht. Bevorzugt handelt es sich bei der Kenngröße um eine geometrische Eigenschaft und/oder eine optische Eigenschaft des optischen Instruments. Bei der Kenngröße handelt es sich beispielsweise um eine Information zu zumindest einem von einem Krümmungsradius, einer Vergrößerung, einem Material, einer Oberflächenbeschaffenheit und/oder einem Herstellungsprozess und/oder zu einer Eigenschaft der Halterung des optischen Instruments.

Schließlich erfolgt in dem erfindungsgemäßen Verfahren gemäß der vorliegenden Offenbarung ein Auswählen eines optischen Instruments aus einer Mehrzahl vorbekannter optischer Instrumente mit vorbekannten Kenngrößen anhand der ermittelten Kenngröße. Beispielsweise ist das OCT-System und bevorzugt das Operationsmikroskop mit einer Mehrzahl in den Strahlengang einbringbarer optischer Instrumente ausgestattet oder erweiterbar. In diesem Fall ermöglicht bereits eine ungefähr ermittelte Kenngröße des optischen Instruments im Verfahren gemäß der vorliegenden Offenbarung eine ausreichende Klassifizierung des optischen Instruments und somit auch eine eindeutige Zuordnung zu einem der Mehrzahl vorbekannter optischer Instrumente. Somit erfolgt eine Identifikation eines aktuell im Strahlengang befindlichen optischen Instruments der Mehrzahl vorbekannter optischer Instrumente vorteilhaft mit hoher Sicherheit. Eine Nutzereingabe zur Auswahl eines der Mehrzahl optischer Instrumente ist somit vorteilhaft korrigierbar oder vollständig ersetzbar.

Das erfindungsgemäße Verfahren ermöglicht vorteilhaft ein automatisiertes Identifizieren von in einen Strahlengang eines OCT-Systems und bevorzugt eines Operationsmikroskops eingebrachten optischen Instrumenten anhand einer automatisierten Auswertung von mit dem OCT-System erfassten OCT-Signalen. Das Verfahren gemäß der vorliegenden Offenbarung ermöglicht ebenso vorteilhaft eine Klassifizierung des optischen Instruments, welche wiederrum ausreichend zur Auswahl eines einer Mehrzahl vorbekannter optischer Instrumente ist. Mit dem Verfahren gemäß der vorliegenden Offenbarung können somit Eingabe- und Bedienfehler zur Auswahl in den Strahlengang eines OCT-Systems eingebrachter optischer Instrumente vorteilhaft vermieden oder deren Auswirkungen reduziert werden.

In einer bevorzugten Durchführungsform des Verfahrens gemäß der vorliegenden Offenbarung wird das OCT-Signal basierend auf einem von einer Oberfläche des optischen Instruments rückgestreuten Lichtsignals erfasst. Dies ermöglicht vorteilhaft das Ermitteln geometrischer und/oder optischer Eigenschaften der Oberfläche des optischen Instruments. Mit anderen Worten umfasst das Erfassen des OCT-Signals dabei das Erfassen von durch die Oberfläche des optischen Instruments gestreuten beziehungsweise zumindest teilreflektierten Anteilen eines Probenstrahls. Anhand des so erfassten OCT-Signals wird ferner bevorzugt eine optische Eigenschaft, beispielsweise die Oberflächenrauigkeit, und/oder eine geometrische Eigenschaft, beispielsweise die Form, der Oberfläche des optischen Instruments ermittelt. Wie obenstehend beschrieben, kann dies anhand des OCT-Signals selbst und/oder durch Auswertung eines anhand des OCT-Signals erstellten OCT-Bildes erfolgen.

In einer besonders bevorzugten Durchführungsform des Verfahrens gemäß der vorliegenden Offenbarung werden ein oder mehrere OCT-Signale basierend auf von einer ersten Oberfläche und/oder von einer zweiten Oberfläche des optischen Instruments rückgestreuten Lichtsignalen erfasst. Beispielsweise wird ein erstes OCT-Signal basierend auf von einer ersten Oberfläche des optischen Instruments rückgestreuten Lichtsignalen und wird ein zweites OCT-Signal basierend auf von einer zweiten Oberfläche des optischen Instruments rückgestreuten Lichtsignalen erfasst. Das erste OCT-Signal und das zweite OCT-Signal werden bevorzugt mit zumindest einem A-Scan erfasst. Anhand der erfassten OCT-Signale werden dann eine Form der ersten Oberfläche und/oder eine Form der zweiten Oberfläche ermittelt. Ferner wird vorteilhaft eine Dicke des optischen Instruments, also ein Abstand der ersten Oberfläche und der zweiten Oberfläche, anhand der OCT-Signale ermittelt. Bei dem Abstand handelt es sich bevorzugt um einen größten Abstand der beiden gegebenenfalls gekrümmten Oberflächen, also um den Abstand an einem dicksten Punkt des optischen Instruments. Wird lediglich ein einziger A-Scan durchgeführt, erfolgt dieser bevorzugt an einem zuvor in einem Videobild identifiziertem Punkt, beispielsweise am Mittelpunkt eines in dem Videobild rotationssymmetrisch erscheinenden optischen Instruments (Draufsicht auf eine Lupe). Schließlich wird die Kenngröße des optischen Instruments anhand der Form zumindest einer der Oberflächen und/oder anhand der Dicke des optischen Instruments ermittelt.

In einer besonders bevorzugten Durchführungsform des Verfahrens gemäß der vorliegenden Offenbarung wird anhand der Formen der Oberflächen und der Dicke des optischen Instruments eine optische Transferfunktion des optischen Instruments ermittelt. Die optische Transferfunktion ist dabei eine zur Beschreibung der Abbildungs- bzw. Wiedergabequalität des optischen Instruments sowie der Güte der (Punkt-)Bildübertragung verwendete Gütefunktion. Mit anderen Worten wird anhand der Formen und der Dicke eine Abbildungsfunktion des optischen Instruments ermittelt. Ein Aspekt der optischen Transferfunktion ist beispielsweise die Vergrößerung durch das optische Instrument. Weitere Aspekte der optischen Transferfunktion betreffen beispielsweise die Punktspreizfunktion der Abbildung, beispielsweise aufgrund von charakteristischen Abbildungsfehlern des optischen Instruments. Die optische Transferfunktion, OTF, ist formal als Fourier-Transformierte der Punktspreizfunktion, PSF, (Impulsantwort der Optik, Bild einer Punktquelle) definiert und als Fourier-Transformation komplexwertig. Im üblichen Fall einer um ihr Zentrum symmetrischen PSF ist die OTF reellwertig. Die Modulationsübertragungsfunktion, MTF, wird formal als der absolute Wert der komplexen OTF definiert und vernachlässigt somit Phaseneffekte der OTF.

Verschiedenen optischen Instrumenten sind verschiedene Abbildungsfunktionen zuordenbar, so dass anhand der ermittelten Abbildungsfunktion auf ein bestimmtes optisches Instrument geschlossen werden kann. Zudem ist die Kenntnis der Abbildungsfunktion vorteilhaft an sich bereits ausreichend für nachfolgende Verwendungen, selbst ohne eine Identifikation des optischen Instruments oder eines Typs des optischen Instruments. Diese Durchführungsform ist somit besonders vorteilhaft auf nicht vorbekannte optische Instrumente anwendbar, beispielsweise optische Instrumente eines Herstellers, der vom Hersteller eines Operationsmikroskops abweicht.

In einer ferner bevorzugten Durchführungsform des Verfahrens gemäß der vorliegenden Offenbarung wird zumindest ein Linienscan zum Erfassen einer Mehrzahl von OCT-Signalen durchgeführt. Es erfolgt somit ein Abrastern des optischen Instruments sowohl in einer Tiefenrichtung (beispielsweise entlang einer optischen Achse des OCT-Systems und bevorzugt eines Operationsmikroskops) als auch in einer lateralen Richtung (beispielsweise quer zu einer optischen Achse des OCT-Systems und bevorzugt eines Operationsmikroskops). Mit anderen Worten wird zumindest ein B-Scan des optischen Instruments durchgeführt. Die Eigenschaft und/oder die Position des optischen Instruments werden dann bevorzugt anhand der Mehrzahl von OCT-Signalen ermittelt. Vorteilhaft sind gemäß dieser Durchführungsform besonders geometrische Eigenschaften mit hoher Güte ermittelbar. Eine geometrische Eigenschaft (Form) der Oberfläche des optischen Instruments umfasst gemäß dieser Durchführungsform bevorzugt zumindest eine Schnittline der Oberfläche des optischen Instruments und mithin bevorzugt eine Ausdehnung, eine Krümmung, eine Wölbung und/oder einen Radius des optischen Instruments. Bei der anhand der geometrischen Eigenschaft (Form) der Oberfläche des optischen Instruments ermittelten Kenngröße des optischen Instruments handelt es sich bevorzugt um einen Krümmungsradius der Oberfläche des optischen Instruments. Anhand des Krümmungsradius ist das optische Instrument vorteilhaft identifizierbar beziehungsweise hinreichend bestimmt. Vorteilhaft ist anhand der Form nur einer Oberfläche der Krümmungsradius und somit das optische Instrument ermittelbar, bei gleichzeitiger geringer Erfassungszeit des OCT-Signals.

In einer besonders bevorzugten Durchführungsform wird eine Mehrzahl von Linienscans durchgeführt. Mit anderen Worten wird eine Mehrzahl von B-Scans des optischen Instruments durchgeführt. Die Eigenschaft und/oder Position, insbesondere die geometrische Eigenschaft (Form der Oberfläche), des optischen Instruments wird dann bevorzugt anhand der Mehrzahl von Linienscans (B-Scans) ermittelt. Diese Durchführungsform ist auf eine oder mehrere Oberflächen des optischen Instruments anwendbar. Bevorzugt sind die Scanrichtungen der Mehrzahl von Linienscans dabei zueinander jeweils um einen ebenen Winkel rotiert, bevorzugt um einen zur Tiefenrichtung des optischen Instruments (des Strahlengangs des OCT-Systems, bevorzugt des Operationsmikroskops) senkrechten Winkel. Alternativ oder zusätzlich sind die Scanrichtungen der Mehrzahl von Linienscans dabei zueinander in lateraler Richtung (quer zur Tiefenrichtung) verschoben. Mit anderen Worten werden bevorzugt mehrere B-Scans in einer zur optischen Achse des OCT-Systems senkrechten Ebene durchgeführt. Mit dieser Durchführungsform ist eine Oberflächenform eines optischen Instruments vorteilhaft mit besonders hoher Genauigkeit ermittelbar. Dies erhöht gleichsam vorteilhaft die Genauigkeit einer anhand der Form ermittelten optischen Transferfunktion, eine Identifikation des optischen Instruments und/oder eine Bilderzeugung.

In einer Variante des erfindungsgemäßen Verfahrens wird die Position des optischen Instruments anhand von zumindest einem OCT-Signal ermittelt. Das zumindest eine OCT-Signal entsteht (beispielsweise als A-Scan) durch Abrastern der Tiefenrichtung (entlang der optischen Achse) des OCT-Systems. Anhand des OCT-Signals ist das optische Instrument mithin entlang dieser Tiefenrichtung (optischen Achse) auffindbar. Bevorzugt wird eine Position des optischen Instruments anhand eines charakteristischen Punkts einer Oberfläche des optischen Instruments, beispielsweise eines Scheitelpunkts, ermittelt. Das Ermitteln eines solchen charakteristischen Punkts erfordert gegebenenfalls wiederum das Durchführen eine Mehrzahl von Linienscans wie obenstehend beschrieben. Die Dicke des optischen Instruments ist jedoch in der Regel vernachlässigbar gegenüber der die Position des optischen Instruments bestimmenden Ausdehnung einer Halterung. Somit ist prinzipiell auch ein A-Scan zum Abschätzen der Position des optischen Instruments ausreichend. Die so ermittelte Position des optischen Instruments ist vorteilhaft beim Ermitteln der optischen Transferfunktion des optischen Instruments und/oder bei dessen Identifikation verwendbar.

Besonders bevorzugt erfolgt gemäß dieser Durchführungsform eine Analyse des zumindest einen OCT-Signals auf das Vorliegen einer Oberfläche des optischen Instruments. Mit anderen Worten wird das zumindest eine OCT-Signal (beispielsweise ein A-Scan) daraufhin analysiert, ob eine Oberfläche oder zumindest ein Oberflächenabschnitt des optischen Instruments darin ermittelbar ist. Ein (Proben-) Strahlengang des OCT-Systems sollte, abgesehen von einem eventuell darin eingebrachten optischen Instrument und der abzubildenden Probe, normalerweise frei von anderen Objekten sein. Somit lässt sich beim Vorliegen einer Oberfläche in dem zumindest einem erfassten OCT-Signal auf ein in den Strahlengang eingebrachtes optisches Instrument schließen, sofern es sich nicht um eine Probenoberfläche handelt. Dies ist, neben der Form, aufgrund unterschiedlicher Reflexionseigenschaften von biologischem Gewebe und optischen Instrumenten auch anhand der OCT-Signalstärke erkennbar. Gemäß dieser Durchführungsform wird die Oberfläche des optischen Instruments in dem zumindest einen OCT-Signal aufgefunden und wird anhand dessen die Position des optischen Instruments ermittelt. Darüber hinaus werden bevorzugt auch weitere OCT-Signale zum Ermitteln der Position des optischen Instruments herangezogen, beispielsweise wenn mehrere OCT-Signale (beispielsweise A-Scans) die Oberfläche des optischen Instruments zumindest teilweise (punktweise) abbilden.

In einer ebenfalls bevorzugten Durchführungsform des Verfahrens gemäß der vorliegenden Offenbarung umfasst eine anhand der Position des optischen Instruments ermittelte Kenngröße des optischen Instruments eine Ausdehnung einer Halterung des optischen Instruments. Besonders bevorzugt handelt es sich dabei um eine Position des optischen Instruments entlang einer Tiefenrichtung des OCT-Scans, sprich auf der optischen Achse eines Probenstrahlengangs des OCT-Systems und bevorzugt eines Operationsmikroskops. Auch die ermittelte Ausdehnung betrifft dabei vorzugsweise eine Ausdehnung entlang dieser optischen Achse des Probenstrahlengangs des OCT-Systems. Verschiedene optische Instrumente weisen in der Regel verschiedene Halterungen mit verschiedenen Ausdehnungen entlang der optischen Achse auf. Indem die Position des optischen Instruments ermittelt wird, kann mittelbar auf diese Ausdehnung der Halterung geschlossen werden, welche dadurch in der Regel eindeutig einem bestimmten optischen Instrument zugeordnet werden kann.

Gemäß einer ebenfalls bevorzugten Durchführungsform kommt eine Swept-Source Quelle als OCT Lichtquelle zum Einsatz. Dies stellt vorteilhaft einen Freiheitsgrad von beispielsweise 30 mm bis 40 mm in der Tiefenmessung bereit und ermöglicht somit vorteilhaft eine weitreichende Rasterung in der Tiefenrichtung des optischen Instruments beziehungsweise des Strahlengangs des OCT-Systems und bevorzugt des Operationsmikroskops. Somit kann vorteilhaft eine Ausdehnung einer Halterung anhand eines OCT-Signals ermittelt werden.

In einer ebenfalls bevorzugten Durchführungsform des erfindungsgemäßen Verfahrens wird anhand des OCT-Signals eine optische Eigenschaft des optischen Instruments ermittelt und umfasst die optische Eigenschaft zumindest eines von einer Streueigenschaft, einer Dispersionseigenschaft, einem Reflexionsvermögen, einer Materialeigenschaft und/oder einer Beschichtung des optischen Instruments. Das zur Herstellung des optischen Instruments verwendete Material (beispielsweise Kunststoff oder Glas) und/oder das verwendete Herstellungsverfahren (beispielsweise Spritzgießen oder Schleifen) und/oder eine verwendete Oberflächenbeschichtung beeinflussen eine Oberflächenbeschaffenheit beziehungsweise - güte des optischen Instruments sowie Streueigenschaften, Dispersionseigenschaften und Reflexionsvermögen des optischen Instruments. Insbesondere die Streueigenschaften, Dispersionseigenschaften und Reflexionsvermögen beeinflussen das OCT-Signal unmittelbar und sind somit anhand dessen ermittelbar. Anhand des OCT-Signals kann mithin auf eine Materialeigenschaft, einer Beschichtung und/oder ein Herstellungsverfahren des optischen Instruments geschlossen werden. Anhand dieser Informationen ist vorteilhaft eine Kenngröße des optischen Instruments ermittelbar und mithin vorteilhaft das optische Instrument selbst.

In dem erfindungsgemäßen Verfahren handelt es sich bei der Struktur um eine in dem Strahlengang angeordnete anatomische Struktur. Beispielsweise handelt es sich bei einem ophthalmologischen Eingriff um eine anatomische Struktur des Auges, insbesondere des Augenhintergrundes. Besonders bevorzugt handelt es sich bei der anatomischen Struktur um den Sehnervenkopf, den gelben Fleck (Makula), die Sehgrube (Fovea), Bestandteile der Sehgrube (Umbo, Faveola, Foveal Avascular Zone - FAZ), Randbereiche der Sehgrube (Parafovea, Perifovea) oder um Blutgefäße im Bereich des Augenhintergrundes oder der Netzhaut. Gemäß dieser Durchführungsform handelt es sich bei der ermittelten Eigenschaft der anatomischen Struktur beispielsweise um eine Form, Größe und/oder (Relativ-)Lage der anatomischen Struktur. Bei der ermittelten Eigenschaft der Struktur handelt es sich gemäß dieser Durchführungsform beispielsweise um den Durchmesser der kreisförmigen Fovea in dem erfassten OCT-Signal. Gemäß der Erfindung wird ferner die Kenngröße des optischen Instruments anhand eines Vergleichs der ermittelten Eigenschaft der anatomischen Struktur mit einer Referenzinformation zu der anatomischen Struktur ermittelt wird. Dabei betreffen auch diese Referenzinformationen bevorzugt eine Größe und/oder eine Form der anatomischen Struktur. Mit anderen Worten wird die Struktur zunächst anhand der a priori vorliegenden Informationen zu der vorbestimmten Struktur ermittelt, beispielsweise als runde, vertiefte Struktur im Augenhintergrund (Fovea), wird anschließend eine Größe und/oder Form der Struktur ermittelt und mit der diesbezüglichen Referenzinformation zu Größe und/oder Form verglichen. Anhand des Vergleichs lässt sich auf eine Abbildung durch ein in den Strahlengang des OCT-Systems eingebrachtes optisches Instrument und folglich auf das Instrument selbst schließen.

Bevorzugt erfolgt in einer Durchführungsform des Verfahrens gemäß der vorliegenden Offenbarung zunächst eine Auswahl (Nutzerauswahl) eines optischen Instruments aus der Mehrzahl vorbekannter optischer Instrumente mittels einer Nutzereingabe über eine Nutzerschnittstelle. Dies ist beispielsweise eine Auswahl wie im Stand der Technik bekannt. Diese Auswahl (Nutzerauswahl) erfolgt beispielsweise zu einem beliebigen Zeitpunkt nachdem eine Auswahl anhand der ermittelten Kenngröße wie vorbeschrieben erfolgt ist. Alternativ erfolgt diese Auswahl (Nutzerauswahl) vor einer Auswahl anhand der ermittelten Kenngröße und/oder löst diese Auswahl (Nutzerauswahl) bevorzugt die Durchführung der Schritte des Verfahrens gemäß der vorliegenden Offenbarung aus. Ferner bevorzugt wird über ein Ausgabemittel ein Hinweis an den Nutzer ausgegeben, wenn die Auswahl anhand der Nutzereingabe von der Auswahl anhand der ermittelten Kenngröße abweicht. Die Warnung erfolgt bevorzugt über ein Ausgabemittel, wie beispielsweise einen Bildschirm, beispielsweise als ein Pop-Up-Fenster und/oder über einen Lautsprecher als ein Warnton oder dergleichen. Ebenso bevorzugt wird der Hinweis ausgegeben, indem dieser an aufgenommene Bilddaten (Videoaufnahmen oder Bildaufnahmen) angeheftet beziehungsweise in diese eingefügt wird. Somit ist bei einer späteren Auswertung der Bilddaten ersichtlich, dass gegebenenfalls eine Fehlauswahl seitens des Nutzers vorlag. Alternativ oder zusätzlich wird die Auswahl anhand der Nutzereingabe durch die Auswahl anhand der ermittelten Kenngröße ersetzt, sofern eine Abweichung zwischen diesen besteht.

In einer weiteren Durchführungsform des Verfahrens gemäß der vorliegenden Offenbarung ist die ermittelte Kenngröße keinem einer Mehrzahl vorbekannter optischer Instrumente mit vorbekannten Kenngrößen zuordenbar. Beispielsweise ist das OCT-System und bevorzugt das Operationsmikroskop mit einer Mehrzahl in den Strahlengang einbringbarer optischer Instrumente ausgestattet oder erweiterbar. Die ermittelte Kenngröße kann jedoch keinem dieser optischen Instrumente mit einem vordefinierten Gütemaß zugeordnet werden. Eine solche Situation kann beispielsweise auftreten, wenn ein OCT-System und bevorzugt Operationsmikroskop eines ersten Herstellers mit optischen Instrumenten eines anderen Herstellers betrieben wird. In diesem Fall wird bevorzugt ein Hinweis, beispielsweise eine Warnung, an einen Nutzer ausgegeben. Alternativ und/oder zusätzlich wird eine Funktion des OCT-Systems gesperrt, wie beispielsweise eine Funktion zur quantitativen Auswertung der erfassten Bildsignale oder dergleichen.

Ebenfalls bevorzugt wird in einer Durchführungsform des Verfahrens gemäß der vorliegenden Offenbarung das erfasste OCT-Signal und eine Kenngröße des in dem Strahlengang des OCT-Systems befindlichen optischen Instruments zur Bilderzeugung und/oder zur quantitativen Auswertung des erfassten OCT-Signals verwendet. Ebenso bevorzugt wird die ermittelte Kenngröße des in dem Strahlengang des OCT-Systems befindlichen optischen Instruments zur Skalierung des Scanverfahrens für den Probenstrahl des OCT-Systems verwendet. Bei der Kenngröße handelt es sich bevorzugt um die ermittelte Kenngröße des in den Strahlengang des OCT-Systems eingebrachten optischen Instruments. Beispielsweise um eine anhand der Form des optischen Instruments ermittelte optische Transferfunktion des eingebrachten optischen Instruments. Ebenfalls bevorzugt handelt es sich bei der Kenngröße um eine anhand der ermittelten Kenngröße ausgewählte vorbekannte Kenngröße eines vorbekannten optischen Instruments. Dieses vorbekannte Kenngröße ist beispielsweise im Speicher des OCT-Systems hinterlegt.

Die Schritte des Verfahrens gemäß der vorliegenden Offenbarung, wie obenstehend beschrieben, erfolgen bevorzugt während des Erfassens eines OCT-Signals mittels des OCT-Systems kontinuierlich oder in Reaktion auf eine Nutzereingabe. In einer alternativen Durchführungsform erfolgen die Schritte des Verfahrens gemäß der vorliegenden Offenbarung in Reaktion auf das Erkennen des Einbringens eines optischen Instruments in den Strahlengang. Beispielsweise weist das OCT-System und bevorzugt ein Operationsmikroskop eine Halterung für in den Strahlengang einbringbare optische Instrumente auf, ist ein aktueller Zustand dieser Halterung detektierbar und indiziert dieser ob das optische Instrument in den Strahlengang einbracht ist oder nicht (beispielsweise eine in den Strahlengang verschwenkbare Halterung mit Lagesensor). In diesem Fall wird das Verfahren gemäß der vorliegenden Offenbarung bevorzugt durchgeführt, wenn die Halterung in den Strahlengang verschwenkt wird.

In einer ferner bevorzugten Durchführungsform werden die Schritte des Verfahrens gemäß der vorliegenden Offenbarung durchgeführt, wenn während des Erfassens eines zeitlichen Verlaufs eines OCT-Signals mittels des OCT-Systems eine Änderung der Skalierung des erfassten OCT-Signals ermittelt wird. Dies wird bevorzugt durch kontinuierliche OCT-Bilderkennung realisiert, wodurch eine plötzliche Änderung einer Größe einer erkannten Struktur feststellbar ist. Dabei kann zum Erkennen der Skalierung dieselbe oder eine andere Struktur verwendet werden, als für das Ermitteln der Kenngröße des optischen Instruments.

Ein weiterer Aspekt der vorliegenden Offenbarung betrifft ein OCT-System zur Durchführung des Verfahrens gemäß der vorliegenden Offenbarung. Das OCT-System weist eine breitbandige Lichtquelle auf, bevorzugt einen durchstimmbaren Laser, einen Breitband-Laser, einen Superkontinuum-Laser und/oder einen Ultrakurzpulslaser. Das OCT-System weist ferner ein zum Erzeugen und Überlagern eines Probenstrahls und eines Referenzstrahls ausgebildetes Interferometer auf. Bei dem Interferometer handelt es sich beispielsweise um ein Michelson-, Mach-Zehner- oder Koster-Interferometer, welches bevorzugt einen Strahlteiler zum Erzeugen (und Überlagern) von Proben- und Referenzstrahlen aus dem Licht der breitbandigen Quelle, einen Referenzstrahlengang und einen Probenstrahlengang aufweist. Ferner bevorzugt weist das Interferometer Mittel zum Einstellen einer untersuchten Probentiefe auf. Dabei kann es sich je nach Messmethode um ein Mittel zum Erzeugen eines Gangunterschieds (wie eines im Referenzstrahl verschiebbaren Spiegel bei einer SD-OCT), ein Mittel zum Separieren von Licht eines bestimmten Gangunterschieds (wie ein optisches Gitter bei einer FD-OCT) oder um Mittel zum Erzeugen von Licht eines bestimmten Gangunterschieds (wie eine durchstimmbaren Quelle bei einer swept source-OCT) handeln. Das OCT-System weist ferner einen zum Erfassen eines durch Überlagerung von Probenstrahl und Referenzstrahl erzeugten Interferenzmusters als zeitaufgelöstes OCT-Signal ausgebildeten Detektor auf. Bei dem Detektor handelt es sich beispielsweise um einen Liniendetektor, ein zweidimensionales Detektorarray, einen Photodetektor, einen dispersiven Detektor, einen CCD-Detektor und/oder einen CMOS-Detektor. Das OCT-System weist ferner einen zum Rastern des Strahlengangs des OCT-Systems ausgebildeten Scanmechanismus auf. Der Scanmechanismus ist bevorzugt dazu ausgebildet, den Probenstrahl in zwei Dimensionen über die Probe zu rastern. Bevorzugt handelt es sich bei dem Scanmechanismus um einen Scanspiegel, alternativ um andere Scanmechanismen, wie beispielsweise einen Glasfaserscanner, einen Prismenscanner, einen Palmer-Scanner oder dergleichen.

Das OCT-System weist ferner eine mit der Lichtquelle und dem Detektor verbundene Steuereinheit auf, wobei die Steuereinheit dazu ausgebildet ist, die Schritte des Verfahrens gemäß der vorliegenden Offenbarung durchzuführen. Die Steuereinheit des OCT-Systems ist insbesondere dazu ausgebildet, die Lichtquelle zum Bestrahlen eines in einen Strahlengang des OCT-Systems eingebrachten optischen Instruments und/oder einer in den Strahlengang des OCT-Systems eingebrachten anatomischen Struktur anzusteuern, den Detektor zum Erfassen eines OCT-Signals des optischen Instruments und/oder der anatomischen Struktur anzusteuern, eine Position des optischen Instruments und/oder eine Eigenschaft der anatomischen Struktur anhand des OCT-Signals zu ermitteln, und eine die Identifikation des optischen Instruments ermöglichenden Kenngröße des optischen Instruments anhand der Position des optischen Instruments und/oder anhand der Eigenschaft der anatomischen Struktur zu ermitteln. Bei der Lichtquelle handelt es sich besonderes bevorzugt um eine Swept-Source Lichtquelle, die vorteilhaft den notwendigen Freiheitsgrad der Tiefenmessung von zumindest 30 mm bis 40 mm bereitstellt. Ein verstellbarer Spiegel im Referenzstrahlengang des Interferometers bei einer Zeitdomain OCT liefert im Gegensatz dazu häufig nur einen Tiefenfreiheitsgrad von weniger als 5 mm.

Ferner bevorzugt weist das OCT-System eine Mehrzahl vorbekannter optischer Instrumente mit vorbekannten Kenngrößen auf, welche bevorzugt eine Auswahl eines dieser optischen Instrumente anhand der ermittelten Kenngröße ermöglichen. Außerdem weist das OCT-System bevorzugt einen Speicher enthaltend Informationen zu einer Mehrzahl vorbekannter optischer Instrumente mit vorbekannten Eigenschaften auf. Ferner bevorzugt weist das OCT-System eine zum Empfangen einer Nutzereingabe ausgebildete Nutzerschnittstelle auf, welche besonders bevorzugt zum Erfassen einer Nutzereingabe zum Auswählen eines optischen Instruments oder zum Starten des Verfahrens gemäß der vorliegenden Offenbarung ausgebildet ist. Das OCT-System weist bevorzugt ein zum Ausgeben eines Hinweises, insbesondere einer Warnung, an den Nutzer ausgebildetes Ausgabemittel auf. Das OCT-System weist ferner bevorzugt ein zum Erfassen eines Bildsignals ausgebildetes operationsmikroskopisches System auf. Ebenso kann das OCT-System in ein operationsmikroskopisches System integriert sein und können die optischen Instruments Teil des operationsmikroskopischen Systems sein. Besonders bevorzugt ist das operationsmikroskopische System gemeinsam mit dem OCT-System in einem multimodalen Operationsmikroskop mit den vorbekannten Komponenten integriert. Im Rahmen dieser Offenbarung wird jedoch zunächst von dem OCT-System ausgegangen.

Weitere bevorzugte Durchführungsformen des Systems gemäß der vorliegenden Offenbarung korrespondieren zu weiteren bevorzugten Ausführungsformen des Verfahrens gemäß der vorliegenden Offenbarung und realisieren dieselben Vorteile wie die Ausführungsformen.

Die Funktionalitäten der erfindungsgemäßen Steuereinheit können durch elektrische oder elektronische Bauteile oder Komponenten (Hardware), durch Firmware (ASIC) implementiert sein und/oder durch Ausführen eines geeigneten Programms (Software) verwirklicht werden. Bevorzugt werden die Funktionalitäten der erfindungsgemäßen Steuereinheit durch eine Kombination von Hardware, Firmware und/oder Software verwirklicht, beziehungsweise implementiert. Beispielsweise sind einzelne Komponenten der erfindungsgemäßen Steuereinheit zum Ausführen einzelner Funktionalitäten als separat integrierter Schaltkreis ausgebildet oder auf einem gemeinsamen integrierten Schaltkreis angeordnet.

Die einzelnen Funktionalitäten der erfindungsgemäßen Steuereinheit sind ferner bevorzugt als ein oder mehrere Prozesse ausgebildet, die auf einem oder mehreren Prozessoren in einem oder mehreren elektronischen Rechengeräten laufen und beim Ausführen von ein oder mehreren Computerprogrammen erzeugt werden. Die Steuereinheit ist dabei dazu ausgebildet, mit den anderen Komponenten, insbesondere der Nutzerschnittstelle, dem bildgebenden Sensor und dem Anzeigemittel zusammenzuarbeiten, um die hierin beschriebenen Funktionalitäten des erfindungsgemäßen Systems zu verwirklichen. Dem Fachmann ist ferner ersichtlich, dass die Funktionalitäten von mehreren Computern (Datenverarbeitungsgeräten, Steuereinheiten, Steuergeräte) kombiniert oder in einem einzigen Gerät kombiniert sein können oder dass die Funktionalität von einem bestimmten Datenverarbeitungsgerät auf eine Vielzahl von Geräten verteilt vorliegen kann, um die Funktionalitäten der erfindungsgemäßen Steuereinheit zu realisieren. Dabei können die Geräte zentral oder dezentral an verschiedenen Orten angeordnet sein. Im Fall einer dezentralen Anordnung sind zwischen den Geräten geeignete Kommunikationsmittel eingerichtet, um Daten zwischen diesen Geräten zu übertragen, die für die Durchführung der jeweiligen Schritte oder für die Bereitstellung der jeweiligen Funktionen der einzelnen Geräte erforderlich sind.

In einer besonders bevorzugten Ausführungsform des erfindungsgemäßen Systems ist dieses in ein Operationsmikroskop integriert. Dabei weist das Operationsmikroskop bevorzugt ein OCT-System wie obenstehend beschrieben auf. Die Steuereinheit des Operationsmikroskops ist bevorzugt als Steuereinheit des erfindungsgemäßen OCT-Systems ausgebildet und insbesondere dazu eingerichtet, anhand von auf einer Speichereinheit des Operationsmikroskops gespeicherten Befehlen das erfindungsgemäße Verfahren durchzuführen. Das Operationsmikroskop weist bevorzugt ein operationsmikroskopisches System mit einem bildgebenden Sensor und einer Optik auf, welche beispielsweise in einer Hauptbeobachterkamera oder einer Umfeldkamera des Operationsmikroskops integriert sind. Das Operationsmikroskop weist ferner bevorzugt zumindest eine Schnittstelle für eine Nutzereingabe und zumindest ein Anzeigemittel auf oder ist zumindest mit diesen verbunden.

Unter einem Operationsmikroskop wird im Rahmen der vorliegenden Offenbarung im weitesten Sinne ein für den Einsatz während einer Operation geeignetes Mikroskop verstanden. Das Operationsmikroskop weist bevorzug eine Halterung auf, die eine Abbildung des Operationsgebiets unabhängig von Kopfbewegungen des Operateurs erlaubt. Ferner bevorzugt weist das Operationsmikroskop zumindest eine Einrichtung zum Aufteilen des Beobachtungsstrahlengangs und zumindest zwei Okulare auf. Alternativ handelt es sich bei dem Operationsmikroskop um ein reines "Digiskop" ohne Okulare. Ebenfalls bevorzugt weist das Operationsmikroskop zumindest einen bildgebenden Sensor auf. Ferner bevorzugt weist das Operationsmikroskop eine Hauptbeobachterkamera und eine Umfeldkamera auf. Das Operationsmikroskop kann kinematische beziehungsweise robotische Hilfsmittel zum Durchführen operativer Eingriffe aufweisen. Alternativ kann ein Operationsmikroskop als medizintechnisches Mikroskop, medizinisch zugelassenes Mikroskop oder medizinisches Mikroskop bezeichnet werden.

Ein weiterer Aspekt betrifft erfindungsgemäß ein Computerprogramm, umfassend Befehle, die bei der Ausführung durch eine Steuereinheit wie obenstehend beschrieben, bevorzugt eines OCT-Systems oder Operationsmikroskops wie obenstehend beschrieben, bewirken, dass das OCT-System oder Operationsmikroskop wie obenstehend beschrieben das erfindungsgemäße Verfahren wie obenstehend beschrieben ausführen. Das Computerprogramm umfasst bevorzugt Befehle, die bei der Ausführung durch eine Steuereinheit wie obenstehend beschrieben, bevorzugt eines OCT-Systems oder Operationsmikroskops, bewirken, dass das OCT-System oder Operationsmikroskop wie obenstehend beschrieben, das erfindungsgemäße Verfahren gemäß einer der bevorzugten Durchführungsformen wie obenstehend beschrieben ausführen.

Das erfindungsgemäße Computerprogramm ist dabei bevorzugt in einem flüchtigen Speicher, beispielsweise einem RAM-Element, oder in einem nicht-flüchtigen Speichermedium, wie beispielsweise einer CD-ROM, einem Flash-Speicher oder dergleichen, abgelegt.

Weitere bevorzugte Ausgestaltungen der Erfindung ergeben sich aus den übrigen, in den Unteransprüchen genannten Merkmalen und den nachfolgend erläuterten Figuren. Die verschiedenen in dieser Anmeldung genannten Ausführungsformen der Erfindung sind, sofern im Einzelfall nicht anders ausgeführt, mit Vorteil miteinander kombinierbar.

### Beschreibung der Figuren

Die Erfindung wird nachfolgend in Ausführungsbeispielen anhand der zugehörigen Zeichnungen erläutert. Es zeigen:
- Figur 1: ein schematisches Ablaufdiagram eines Verfahrens gemäß einer Durchführungsform;
- Figur 2: eine schematische Darstellung eines OCT-Systems gemäß einer Ausführungsform;
- Figur 3: eine schematische Darstellung eines OCT-Systems gemäß einer weiteren Ausführungsform;
- Figur 4: eine schematische Darstellung der Ermittlung einer Kenngröße eines optischen Instruments anhand einer geometrischen Eigenschaft des optischen Instruments; und
- Figur 5: eine schematische Darstellung der Ermittlung einer Kenngröße eines optischen Instruments anhand einer Position des optischen Instruments.

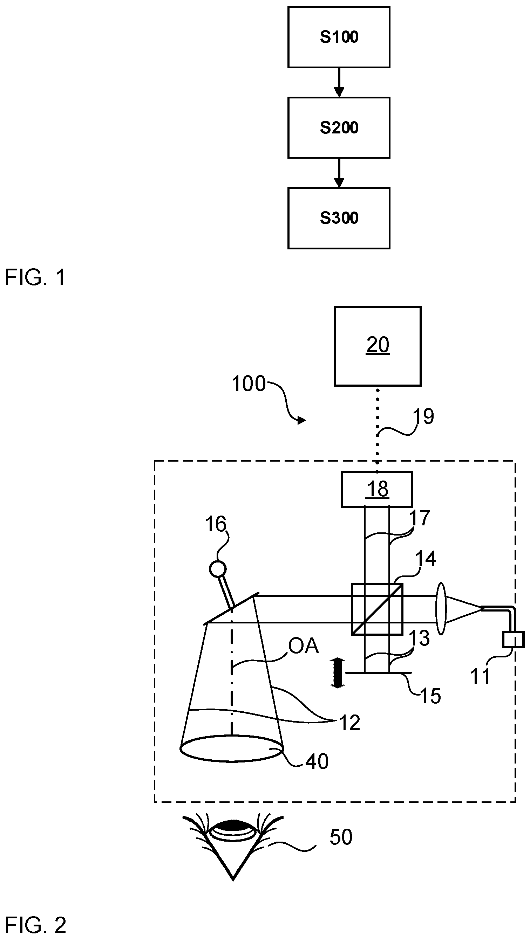

Figur 1 zeigt ein schematisches Ablaufdiagram eines Verfahrens gemäß einer Durchführungsform zum Betrieb eines OCT-Systems 100, beispielsweise des in Figur 2 gezeigten OCT-Systems 100 gemäß einer Ausführungsform. Das Verfahren wird nachfolgend unter Bezugnahme auf das OCT-System 100 der Figur 2 beschrieben.

Das OCT-System 100 weist eine breitbandige Lichtquelle 11 auf, bei der es sich beispielsweise um eine Superlumineszenzdiode oder einen durchstimmbaren Laser handelt. Das Licht der breitbandigen Lichtquelle 11 wird in ein Interferometer mit einem Strahlteiler 14 und einem beweglichen Spiegel 15 geleitet. Im Strahlteiler 14 wird das Licht in einen Probenstrahl 12 und in einen Referenzstrahl 13 geteilt. Der Probenstrahl 12 wird mittels eines Scanspiegels 16 gerastert, beispielsweise über ein im Sichtfeld des Probenstrahlengangs angeordnetes Auge eines Patienten 50. Der Referenzstrahl 13 wird auf den beweglichen Spiegel 15 gelenkt und von diesem zurück auf den Strahlteiler 14 reflektiert. Der Probenstrahl 12 interagiert mit dem Auge des Patienten 50, wird von dort zu dem Scanspiegel 16 zurückgestreut und von diesem auf den Strahlteiler 14 gelenkt. Dort werden der zurückgestreute Probenstrahl 12 und der reflektierte Referenzstrahl 13 zur Überlagerung gebracht, wobei ein Gangunterschied zwischen den überlagerten Strahlen 12, 13 durch den beweglichen Spiegel 15 eingestellt wird. Das so erzeugte Interferenzmuster 17 wird mittels eines Detektors 18, beispielsweise eines CCD-Detektors oder eines CMOS-Detektors, erfasst und als OCT-Signal 19 an eine Steuereinheit 20 übermittelt, wobei die Steuereinheit 20 zum Durchführen des offenbarten Verfahrens ausgebildet ist.

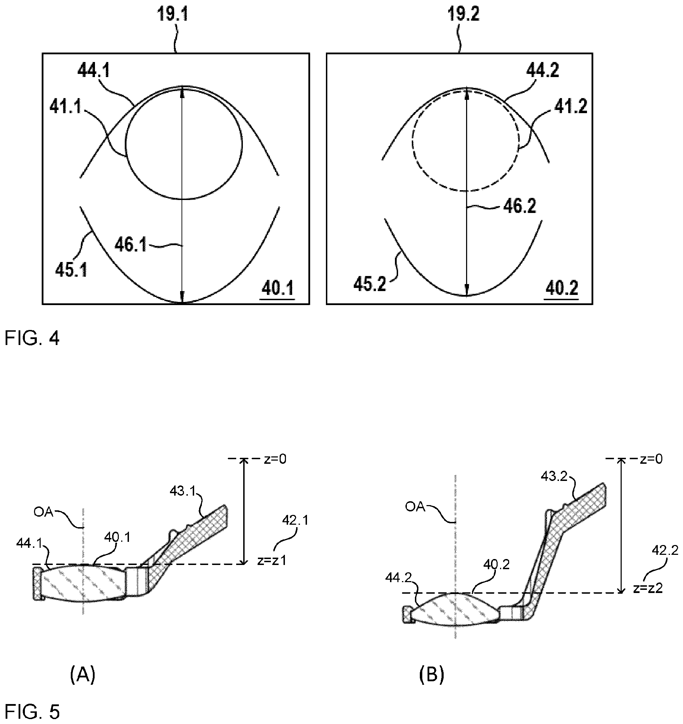

Beispielsweise mittels dieses OCT-Systems 100 erfolgt in einem ersten Schritt des Verfahrens erfindungsgemäß das Erfassen S100 eines OCT-Signals 19 eines in den Strahlengang 12 des OCT-Systems 10 eingebrachten optischen Instruments 40. Mit anderen Worten wird der Probenstrahl 12 von dem optischen Instrument 40, beispielsweise einer Oberfläche desselben, zurückgestreut und wird der zurückgestreute Anteil in dem Interferometer 14 mit dem Referenzstrahl 13 zur Überlagerung gebracht. Ein durch diese Überlagerung erzeugtes Referenzmuster 17 wird vom Detektor 18 als OCT-Signal 19 erfasst. In einem zweiten Schritt S200 des Verfahrens gemäß der vorliegenden Offenbarung erfolgt das Ermitteln einer Eigenschaft, beispielsweise einer geometrischen Eigenschaft 41, des optischen Instruments 40, wie in Figur 4 dargestellt, anhand des erfassten OCT-Signals 19. Alternativ oder zusätzlich erfolgt in dem zweiten Schritt S200 das Ermitteln einer Position 42 des optischen Instruments 40, wie in Figur 5 dargestellt, anhand des erfassten OCT-Signals 19. In einem dritten Schritt S300 des Verfahrens gemäß der vorliegenden Offenbarung erfolgt das Ermitteln einer Kenngröße des optischen Instruments 40 anhand der zuvor in Schritt S200 ermittelten Eigenschaft, insbesondere der geometrischen Eigenschaft 41, und/oder anhand der Position 42 des optischen Instruments 40.

Der zweite Schritt S200 und der dritte Schritt S300 werden nachfolgend unter Bezugnahme auf die Figuren 4 und 5 beispielhaft erläutert.

Figur 4 zeigt dabei auf zwei OCT-Signalen 19.1, 19.2 basierende OCT Linienscans (B-Scans) von zwei verschiedenen optischen Instrumenten 40.1 und 40.2, insbesondere von zwei verschiedenen Funduslupen 40.1 und 40.2. Jede der Funduslupen 40.1 und 40.2 weist dabei eine erste Oberfläche 44 und eine zweite Oberfläche 45 auf. Die Oberflächen 44, 45 beider Funduslupen 40.1, 40.2 sind in den anhand der OCT-Signale 19.1, 19.2 ermittelten OCT-Bildern deutlich erkennbar. Anhand einer durch die eingefügten Kreise angedeuteten Bildanalyse wird im zweiten Schritt S200 des Verfahrens gemäß der vorliegenden Offenbarung ein Krümmungsradius 41.1 der ersten Funduslupe 40.1 und ein Krümmungsradius 41.2 der zweiten Funduslupe 40.2 ermittelt. Beispielsweise beträgt der Krümmungsradius 41.1 der ersten Funduslupe 40.1 etwa 6,48 mm und beträgt der Krümmungsradius 41.2 der zweiten Funduslupe 40.2 etwa 5,88 mm. Darüber hinaus wird im zweiten Schritt des Verfahrens S200 anhand der ersten und zweiten Oberflächen 44, 45 in beiden OCT-Bildern eine Dicke 46.1 der ersten Funduslupe 40.1 und eine Dicke 46.2 der zweiten Funduslupe 40.2 ermittelt.

Anhand dieser anhand der OCT-Signale 19.1, 19.2 ermittelten geometrischen Eigenschaften 41, 46 der Funduslupen 40.1, 40.2 wird im dritten Schritt S300 des Verfahrens gemäß der vorliegenden Offenbarung eine Kenngröße der Funduslupen 40.1, 40.2 ermittelt. Insbesondere wird anhand des ersten Krümmungsradius 41.1 und gegebenenfalls anhand der ersten Dicke 46.1 ermittelt, dass die erste Funduslupe 40.1 eine Vergrößerung von etwa 60 Dioptrien aufweist. Anhand des zweiten Krümmungsradius 41.2 und gegebenenfalls anhand der zweiten Dicke 46.2 wird im dritten Schritt S300 des Verfahrens gemäß der vorliegenden Offenbarung ferner ermittelt, dass die zweite Funduslupe 40.2 eine Vergrößerung von etwa 128 Dioptrien aufweist. Diese in Schritt S300 ermittelten Kenngrößen der Funduslupen 40.1, 40.2 sind vorteilhaft unmittelbar zur Bilderzeugung und/oder Skalierung weiterer mit dem OCT-System 100 erfasster OCT-Signale 19 und/oder zur Skalierung des Scanverfahrens für den Probenstrahl 12 des OCT-Systems 100 verwendbar. Die in Schritt S300 ermittelten Kenngrößen der Funduslupen 40.1, 40.2 sind ebenso zur Klassifizierung beziehungsweise Erkennung der Funduslupen 40.1, 40.2 als bestimmte Funduslupen einer Mehrzahl vorbekannter Funduslupen des OCT-Systems 100 verwendbar.

Figur 5 zeigt zwei schematische Darstellungen von optischen Instrumenten 40.1 und 40.2, welche das Ermitteln von Kenngrößen der optischen Instrumente 40.1, 40.2 anhand von Positionen 42.1, 42.2 der optischen Instrumente 40.1, 40.2 erlauben. Dabei weist jedes der optischen Instrumente 40.1, 40.2, insbesondere der Funduslupen 40.1, 40.2, eine erste Oberfläche 44.1, 44.2 auf. Zudem weist jede der Funduslupen 40.1, 40.2 eine Halterung 43.1, 43.2 auf, mit der die Funduslupen 40.1, 40.2 an nicht dargestellten Operationsmikroskopen befestigt sind. Im zweiten Schritt S200 des Verfahrens gemäß der vorliegenden Offenbarung werden die Positionen der Funduslupen 40.1, 40.2 in einer Tiefenrichtung z entlang einer optischen Achse OA dieser Operationsmikroskope ermittelt.

Dazu wird zumindest ein OCT-Signal erfasst (also beispielsweise ein A-Scan durchgeführt), bevorzugt wird zumindest ein Linienscan (B-Scan), wie in Figur 4 dargestellt, durchgeführt. Das zumindest eine OCT-Signal, bevorzugt der Linienscan, beziehungsweise ein anhand dessen erzeugtes OCT-Bild wird auf das Vorliegen einer ersten Oberfläche 44.1, 44.2 der jeweiligen Funduslupe 40.1, 40.2 analysiert. Wird eine erste Oberfläche 44.1, 44.2 der jeweiligen Funduslupe 40.1, 40.2 ermittelt, in einem Linienscan insbesondere ein Scheitelpunkt der jeweiligen ersten Oberfläche 44.1, 44.2, wird in dem zweiten Schritt S200 des Verfahrens gemäß der vorliegenden Offenbarung anhand der Lage der ersten Oberflächen 44.1, 44.2 ermittelt, dass die erste Funduslinse 40.1 an einer ersten Position 42.1 entlang der optischen Achse OA angeordnet ist, insbesondere bei z = z1, und dass die zweite Funduslupe 40.2 an einer zweiten Position 42.2 entlang der optischen Achse OA angeordnet ist, insbesondere bei z = z2.

Anhand der so ermittelten Positionen 42.1, 42.2 der Funduslupen 40.1, 40.2 entlang der optischen Achse OA wird nachfolgend im Schritt S300 auf die Höhe von Halterungen 43.1, 43.2 der Funduslupen 40.1, 40.2 als die Ausdehnungen dieser Halterungen 43.1, 43.2 in z-Richtung beziehungsweise entlang der optischen Achse OA geschlossen. Dabei stellt die Höhe der Halterungen 43.1, 43.2 eine Kenngröße für die optischen Instrumente 40.1, 40.2 dar, anhand derer diese optischen Instrumente 40.1, 40.2 eindeutig identifizierbar sind, beispielsweise als Auswahl aus einer Mehrzahl vorbekannter optischer Instrumente.

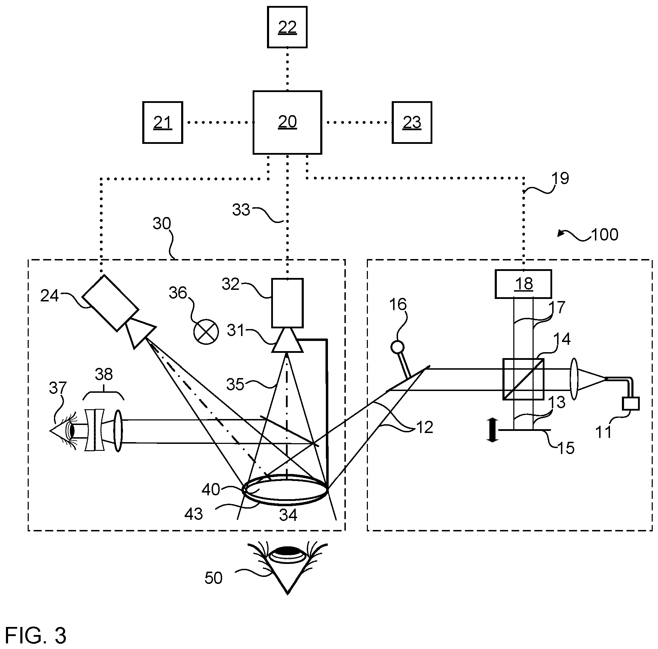

Figur 3 zeigt eine schematische Darstellung eines OCT-Systems 100 gemäß einer weiteren Ausführungsform. Das OCT-System 100 weist dabei unter anderem dieselben Komponenten auf, wie das mit Bezug zu Figur 2 beschriebene OCT-System 100. Auf eine wiederholte Beschreibung dieser Komponenten wird an dieser Stelle verzichtet. Ferner weist das OCT-System 100 der Figur 3 ein operationsmikroskopisches System 30 auf, welches zum Erfassen eines Bildsignals 33 von einem Sichtfeld 34 ausgebildet ist, wobei dieses Sichtfeld 34 sowohl das Auge des Patienten 50 als auch das optische Instrument 40 umfassen kann. Ebenso kann ein operationsmikroskopisches System ein OCT-System 100 aufweisen (nicht dargestellt).

Das operationsmikroskopische System 30 weist dabei zumindest einen zum Erfassen des Bildsignals 33 ausgebildeten bildgebenden Sensor 32 und eine Optik 31 auf. Bildgebender Sensor 32 und Optik 31 sind Teil einer Hauptbeobachterkamera des operationsmikroskopischen Systems 30. Das operationsmikroskopische System 30 weist ferner eine Umfeldkamera 24 auf, welche dazu ausgebildet ist, das Auge des Patienten 50 mit einer geringeren Zoomstufe darzustellen, als die Hauptbeobachterkamera. Das operationsmikroskopische System 30 weist ferner einen in Strahlengang 35 des operationsmikroskopischen Systems 30 angeordneten Strahlteiler zum teilweisen Umlenken von Objektstrahlen auf ein Okular 38 und dadurch auf das Auge eines Betrachters 37 auf. Zudem weist das operationsmikroskopische System 30 eine weitere Lichtquelle 36 zum Beleuchten des Auges des Patienten 50 auf. Die Hauptbeobachterkamera sendet Bildsignale 33 über eine geeignete Datenverbindung an die Steuereinheit 20, mit der auch die Umfeldkamera 24 verbunden ist. Die Steuereinheit 20 ist mit einem Speicher 21 verbunden, auf dem beispielsweise Algorithmen zur Bilderkennung und vorbekannte Kenngrößen vorbekannter optischer Instrumente gespeichert sind. Die Steuereinheit 20 ist ferner mit einer Nutzerschnittstelle 22 zum Erfassen einer Nutzereingabe und mit einem Ausgabemittel 23 zur Ausgabe eines Warnhinweises verbunden. Figur 3 zeigt ferner eine Halterung 43 des optischen Instruments 40 mit dem dieses an Optik 31 der Hauptbeobachterkamera fixiert ist.

### Bezugszeichenliste

- 100: OCT-System
- 11: breitbandige Lichtquelle
- 12: Probenstrahl
- 13: Referenzstrahl
- 14: Strahlteiler (Interferometer)
- 15: beweglicher Spiegel (Interferometer)
- 16: Scanmechanismus (Scanspiegel)
- 17: Interferenzmuster
- 18: Detektor
- 19: OCT-Signal
- OA: optische Achse
- 20: Steuereinheit
- 21: Speicher
- 22: Nutzerschnittstelle
- 23: Ausgabemittel
- 24: Umfeldkamera
- 30: operationsmikroskopisches System
- 31: Optik
- 32: bildgebender Sensor
- 33: Bildsignal
- 34: Sichtfeld
- 35: Strahlengang
- 36: Lichtquelle
- 37: Auge eines Betrachters
- 38: Okular
- 40: optisches Instrument/Ophthalmoskopierlupe
- 41: geometrische Eigenschaft
- 42: Position
- 43: Halterung
- 44: erste Oberfläche
- 45: zweite Oberfläche
- 46: Dicke
- 50: Auge eines Patienten
- d: Abstand zwischen optischem Instrument und Auge

## Patentansprüche

1. Verfahren einer Steuereinheit (20) eines OCT-Systems (100) zum Betrieb des OCT-Systems (100), das Verfahren aufweisend die Verfahrensschritte:
Erfassen (S100), mittels des OCT-Systems (100), eines OCT-Signals (19) eines in einen Strahlengang (12) des OCT-Systems (10) eingebrachten optischen Instruments (40);
Ermitteln (S200) einer Position (42) des optischen Instruments (40) als Raumlage des optischen Instruments (40) in einem Koordinatensystem des OCT-Systems (100) anhand des OCT-Signals (19);
Ermitteln (S300) einer die Identifikation des optischen Instruments ermöglichenden Kenngröße des optischen Instruments (40) anhand der Position (42) des optischen Instruments (40); und
Identifizieren des optischen Instruments (40) aus einer Mehrzahl vorbekannter optischer Instrumente mit vorbekannten Kenngrößen anhand der ermittelten Kenngröße.

2. Verfahren einer Steuereinheit (20) eines OCT-Systems (100) zum Betrieb des OCT-Systems (100), das Verfahren aufweisend die Verfahrensschritte:
Erfassen (S100), mittels des OCT-Systems (100), eines OCT-Signals (19) einer in den Strahlengang (12) des OCT-Systems (10) eingebrachten anatomischen Struktur durch ein in den Strahlengang des OCT-Systems (100) eingebrachtes optisches Instrument (40);
Ermitteln (S200) einer Eigenschaft der anatomischen Struktur anhand des OCT-Signals (19);
Ermitteln (S300) einer die Identifikation des optischen Instruments ermöglichenden Kenngröße des optischen Instruments (40) anhand eines Vergleichs der ermittelten Eigenschaft der anatomischen Struktur mit einer Referenzinformation zu der anatomischen Struktur; und
Identifizieren des optischen Instruments (40) aus einer Mehrzahl vorbekannter optischer Instrumente mit vorbekannten Kenngrößen anhand der ermittelten Kenngröße.

3. Verfahren nach Anspruch 1, wobei anhand des OCT-Signals (19) ferner eine geometrische Eigenschaft des optischen Instruments (40) ermittelt wird und die Kenngröße des optischen Instruments (40) ferner anhand der geometrischen Eigenschaft des optischen Instruments (40) ermittelt wird.

4. Verfahren nach Anspruch 3,
wobei OCT-Signale (19) basierend auf von einer ersten Oberfläche (44) und/oder von einer zweiten Oberfläche (45) des optischen Instruments (40) rückgestreuten Lichtsignalen erfasst werden,
wobei eine Form der ersten Oberfläche (44) und/oder eine Form der zweiten Oberfläche (45) sowie einer Dicke (46) des optischen Instruments (40) anhand der OCT-Signale (19) ermittelt werden und
wobei die Kenngröße des optischen Instruments (40) anhand der Form zumindest einer der Oberflächen (44, 45) sowie anhand der Dicke (46) des optischen Instruments (40) ermittelt wird.

5. Verfahren nach Anspruch 4, wobei eine optische Transferfunktion des optischen Instruments (40) anhand der Form zumindest einer der Oberflächen (44, 45) sowie anhand der Dicke (46) des optischen Instruments (40) ermittelt wird.

6. Verfahren nach einem der Ansprüche 3 bis 5,
wobei zumindest ein Linienscan zum Erfassen einer Mehrzahl von OCT-Signalen (19) durchgeführt wird und
wobei die geometrische Eigenschaft und/oder Position des optischen Instruments (40) anhand der Mehrzahl von OCT-Signalen (19) ermittelt wird.

7. Verfahren nach Anspruch 6, wobei eine Mehrzahl von Linienscans durchgeführt wird und wobei Scanrichtungen der Mehrzahl von Linienscans zueinander jeweils um einen ebenen Winkel rotiert sind und/oder in lateraler Richtung verschoben sind.

8. Verfahren nach Anspruch 1, wobei eine anhand der Position (42) des optischen Instruments (40) ermittelte Kenngröße des optischen Instruments (40) eine Ausdehnung einer Halterung (43) des optischen Instruments (40) umfasst.

9. Verfahren nach Anspruch 1, wobei anhand des OCT-Signals (19) ferner eine optische Eigenschaft des optischen Instruments (40) ermittelt wird und die optische Eigenschaft (41) zumindest eines von einer Streueigenschaft, einer Dispersionseigenschaft, einem Reflexionsvermögen, einer Materialeigenschaft und/oder einer Beschichtung des optischen Instruments (44) umfasst.

10. Verfahren nach Anspruch 1, ferner aufweisend:
Auswahl eines optischen Instruments (40) aus der Mehrzahl vorbekannter optischer Instrumente mittels einer Nutzereingabe über eine Nutzerschnittstelle (22);
Ausgabe eines Hinweises an den Nutzer, wenn die Auswahl anhand der Nutzereingabe von dem identifizierten optischen Instrument (40) gemäß Anspruch 1 abweicht; und/oder
Ersetzen der Auswahl anhand der Nutzereingabe durch das identifizierte optische Instrument (40) gemäß Anspruch 1.

11. Verfahren nach einem der vorangehenden Ansprüche, wobei das erfasste OCT-Signal (19) und die ermittelte Kenngröße des in den Strahlengang (12) des OCT-Systems (100) eingebrachten optischen Instruments (40) oder eine anhand der ermittelten Kenngröße ausgewählte vorbekannte Kenngröße eines vorbekannten optischen Instruments zur Bilderzeugung und/oder zur quantitativen Auswertung des OCT-Signals (19) verwendet werden.

12. Verfahren nach einem der vorangehenden Ansprüche, wobei die Schritte nach Anspruch 1 während des Erfassens eines OCT-Signals (19) mittels des OCT-Systems (100) kontinuierlich, in Reaktion auf das Erkennen des Einbringens eines optischen Instruments (40) in den Strahlengang (12) des OCT-Systems (100) oder in Reaktion auf das Erkennen einer Änderung der Skalierung des erfassten OCT-Signals (19) in einem zeitlichen Verlauf des OCT-Signals (19) erfolgen.

13. OCT-System (100), aufweisend:
eine breitbandige Lichtquelle (11), bevorzugt einen durchstimmbaren Laser;
ein zum Erzeugen und Überlagern eines Probenstrahls (12) und eines Referenzstrahls (13) ausgebildetes Interferometer (14);
einen zum Erfassen eines durch Überlagerung von Probenstrahl (12) und Referenzstrahl (13) erzeugten Interferenzmusters (17) als zeitaufgelöstes OCT-Signal (19) ausgebildeten Detektor (18);
einen zum Rastern des Strahlengangs (12) des OCT-Systems (100) ausgebildeten Scanmechanismus (16); und
eine mit der Lichtquelle (11) und dem Detektor (18) verbundenen Steuereinheit (20), wobei die Steuereinheit (20) dazu ausgebildet ist:
die Lichtquelle (11) zum Bestrahlen eines in einen Strahlengang (12) des OCT-Systems (100) eingebrachten optischen Instruments (40) anzusteuern;
den Detektor (18) zum Erfassen eines OCT-Signals (19) des optischen Instruments (40) anzusteuern;
eine Position des optischen Instruments (40) als Raumlage des optischen Instruments (40) in einem Koordinatensystem des OCT-Systems (100) anhand des OCT-Signals (19) zu ermitteln,
eine die Identifikation des optischen Instruments ermöglichende Kenngröße des optischen Instruments (40) anhand der Position des optischen Instruments (40) zu ermitteln, und
das optische Instrument (40) aus einer Mehrzahl vorbekannter optischer Instrumente mit vorbekannten Kenngrößen anhand der ermittelten Kenngröße zu identifizieren.

14. OCT-System (100), aufweisend:
eine breitbandige Lichtquelle (11), bevorzugt einen durchstimmbaren Laser;
ein zum Erzeugen und Überlagern eines Probenstrahls (12) und eines Referenzstrahls (13) ausgebildetes Interferometer (14);
einen zum Erfassen eines durch Überlagerung von Probenstrahl (12) und Referenzstrahl (13) erzeugten Interferenzmusters (17) als zeitaufgelöstes OCT-Signal (19) ausgebildeten Detektor (18);
einen zum Rastern des Strahlengangs (12) des OCT-Systems (100) ausgebildeten Scanmechanismus (16); und
eine mit der Lichtquelle (11) und dem Detektor (18) verbundenen Steuereinheit (20), wobei die Steuereinheit (20) dazu ausgebildet ist:
die Lichtquelle (11) zum Bestrahlen einer in den Strahlengang (12) des OCT-Systems (10) eingebrachten anatomischen Struktur anzusteuern;
den Detektor (18) zum Erfassen eines OCT-Signals (19) der anatomischen Struktur durch ein in den Strahlengang des OCT-Systems (100) eingebrachtes optisches Instrument (40) anzusteuern;
eine Eigenschaft der anatomischen Struktur anhand des OCT-Signals (19) zu ermitteln,
eine die Identifikation des optischen Instruments ermöglichende Kenngröße des optischen Instruments (40) anhand eines Vergleichs der ermittelten Eigenschaft der anatomischen Struktur mit einer Referenzinformation zu der anatomischen Struktur zu ermitteln, und
das optische Instrument (40) aus einer Mehrzahl vorbekannter optischer Instrumente mit vorbekannten Kenngrößen anhand der ermittelten Kenngröße zu identifizieren.

15. OCT-System (100) nach Anspruch 13 oder 14, ferner aufweisend:
eine Mehrzahl vorbekannter optischer Instrumente mit vorbekannten Eigenschaften;
einen Speicher (21) enthaltend Informationen zu einer Mehrzahl vorbekannter optischer Instrumente mit vorbekannten Kenngrößen;
eine zum Empfangen einer Nutzereingabe ausgebildete Nutzerschnittstelle (22); und/oder
ein zum Ausgeben einer Warnung an den Nutzer ausgebildetes Ausgabemittel (23).

16. Computerprogramm, umfassend Befehle, die bei der Ausführung durch eine Steuereinheit (20) eines OCT-Systems (100) gemäß Anspruch 13 bewirken, dass das OCT-System (100) ein Verfahren gemäß Anspruch 1 ausführt oder umfassend Befehle, die bei der Ausführung durch eine Steuereinheit (20) eines OCT-Systems (100) gemäß Anspruch 14 bewirken, dass das OCT-System (100) ein Verfahren gemäß Anspruch 2 ausführt.

## Claims

1. Method of a control unit (20) of an OCT system (100) for operating the OCT system (100), the method comprising the following method steps:
capturing (S100), by means of the OCT system (100), an OCT signal (19) of an optical instrument (40) introduced into a beam path (12) of the OCT system (10);
determining (S200) a position (42) of the optical instrument (40) as a spatial pose of the optical instrument (40) in a coordinate system of the OCT system (100) on the basis of the OCT signal (19);
determining (S300) a characteristic variable of the optical instrument (40) enabling the identification of the optical instrument on the basis of the position (42) of the optical instrument (40); and
identifying the optical instrument (40) from a plurality of previously known optical instruments with previously known characteristic variables on the basis of the determined characteristic variable.

2. Method of a control unit (20) of an OCT system (100) for operating the OCT system (100), the method comprising the following method steps:
capturing (S100), by means of the OCT system (100), an OCT signal (19) of an anatomical structure introduced into the beam path (12) of the OCT system (10) by way of an optical instrument (40) introduced into the beam path of the OCT system (100);
determining (S200) a property of the anatomical structure on the basis of the OCT signal (19);
determining (S300) a characteristic variable of the optical instrument (40) enabling the identification of the optical instrument on the basis of a comparison of the determined property of the anatomical structure with reference information relating to the anatomical structure; and
identifying the optical instrument (40) from a plurality of previously known optical instruments with previously known characteristic variables on the basis of the determined characteristic variable.

3. Method according to Claim 1, wherein a geometric property of the optical instrument (40) is furthermore determined on the basis of the OCT signal (19) and the characteristic variable of the optical instrument (40) is furthermore determined on the basis of the geometric property of the optical instrument (40).

4. Method according to Claim 3,
wherein OCT signals (19) are captured on the basis of light signals backscattered from a first surface (44) and/or from a second surface (45) of the optical instrument (40),
wherein a shape of the first surface (44) and/or a shape of the second surface (45) and a thickness (46) of the optical instrument (40) are/is determined on the basis of the OCT signals (19) and
wherein the characteristic variable of the optical instrument (40) is determined on the basis of the shape of at least one of the surfaces (44, 45) and on the basis of the thickness (46) of the optical instrument (40).

5. Method according to Claim 4, wherein an optical transfer function of the optical instrument (40) is determined on the basis of the shape of at least one of the surfaces (44, 45) and on the basis of the thickness (46) of the optical instrument (40).

6. Method according to any of Claims 3 to 5,
wherein at least one line scan is carried out for capturing a plurality of OCT signals (19) and
wherein the geometric property and/or position of the optical instrument (40) are/is determined on the basis of the plurality of OCT signals (19).

7. Method according to Claim 6, wherein a plurality of line scans are carried out and wherein scan directions of the plurality of line scans, with respect to one another, in each case are rotated by a plane angle and/or are displaced in a lateral direction.

8. Method according to Claim 1, wherein a characteristic variable of the optical instrument (40) determined on the basis of the position (42) of the optical instrument (40) comprises an extent of a mount (43) of the optical instrument (40).

9. Method according to Claim 1, wherein an optical property of the optical instrument (40) is furthermore determined on the basis of the OCT signal (19) and the optical property (41) comprises at least one out of a scattering property, a dispersion property, a reflectivity, a material property and/or a coating of the optical instrument (44).

10. Method according to Claim 1, furthermore comprising:
selection of an optical instrument (40) from the plurality of previously known optical instruments by means of a user input via a user interface (22);
output of an indication to the user if the selection on the basis of the user input deviates from the identified optical instrument (40) according to Claim 1; and/or replacing the selection on the basis of the user input with the identified optical instrument (40) according to Claim 1.

11. Method according to any of the preceding claims, wherein the captured OCT signal (19) and the determined characteristic variable of the optical instrument (40) introduced into the beam path (12) of the OCT system (100) or a previously known characteristic variable - selected on the basis of the determined characteristic variable - of a previously known optical instrument are used for image generation and/or for quantitative evaluation of the OCT signal (19).

12. Method according to any of the preceding claims, wherein the steps according to Claim 1 take place during the capturing of an OCT signal (19) by means of the OCT system (100) continuously, in reaction to the detection of the introduction of an optical instrument (40) into the beam path (12) of the OCT system (100) or in reaction to the detection of a change in the scaling of the captured OCT signal (19) in a temporal profile of the OCT signal (19).

13. OCT system (100), comprising:
a broadband light source (11), preferably a tunable laser;
an interferometer (14) designed to generate and superimpose a sample beam (12) and a reference beam (13);
a detector (18) designed to capture an interference pattern (17) generated by superimposition of sample beam (12) and reference beam (13) as a temporally resolved OCT signal (19);
a scanning mechanism (16) designed to scan the beam path (12) of the OCT system (100); and
a control unit (20) connected to the light source (11) and the detector (18), wherein the control unit (20) is designed to:
control the light source (11) for irradiating an optical instrument (40) introduced into a beam path (12) of the OCT system (100);
control the detector (18) for capturing an OCT signal (19) of the optical instrument (40);
determine a position of the optical instrument (40) as a spatial pose of the optical instrument (40) in a coordinate system of the OCT system (100) on the basis of the OCT signal (19),
determine a characteristic variable of the optical instrument (40) enabling the identification of the optical instrument on the basis of the position of the optical instrument (40), and
identify the optical instrument (40) from a plurality of previously known optical instruments with previously known characteristic variables on the basis of the determined characteristic variable.

14. OCT system (100), comprising:
a broadband light source (11), preferably a tunable laser;
an interferometer (14) designed to generate and superimpose a sample beam (12) and a reference beam (13);
a detector (18) designed to capture an interference pattern (17) generated by superimposition of sample beam (12) and reference beam (13) as a temporally resolved OCT signal (19);
a scanning mechanism (16) designed to scan the beam path (12) of the OCT system (100); and
a control unit (20) connected to the light source (11) and the detector (18), wherein the control unit (20) is designed to:
control the light source (11) for irradiating an anatomical structure introduced into the beam path (12) of the OCT system (10);
control the detector (18) for capturing an OCT signal (19) of the anatomical structure by way of an optical instrument (40) introduced into the beam path of the OCT system (100);
determine a property of the anatomical structure on the basis of the OCT signal (19),
determine a characteristic variable of the optical instrument (40) enabling the identification of the optical instrument on the basis of a comparison of the determined property of the anatomical structure with reference information relating to the anatomical structure, and
identify the optical instrument (40) from a plurality of previously known optical instruments with previously known characteristic variables on the basis of the determined characteristic variable.

15. OCT system (100) according to Claim 13 or 14, furthermore comprising:
a plurality of previously known optical instruments with previously known properties;
a memory (21) containing information relating to a plurality of previously known optical instruments with previously known characteristic variables;
a user interface (22) designed to receive a user input; and/or
an output means (23) designed to output a warning to the user.

16. Computer program, comprising instructions which, when executed by a control unit (20) of an OCT system (100) according to Claim 13, cause the OCT system (100) to execute a method according to Claim 1, or comprising instructions which, when executed by a control unit (20) of an OCT system (100) according to Claim 14, cause the OCT system (100) to execute a method according to Claim 2.

## Revendications

1. Procédé d'une unité de commande (20) d'un système OCT (100) pour faire fonctionner le système OCT (100), le procédé comprenant les étapes de procédé suivantes :
l'acquisition (S100), au moyen du système OCT (100), d'un signal OCT (19) d'un instrument optique (40) introduit dans un chemin de faisceau (12) du système OCT (10) ;
la détermination (S200) d'une position (42) de l'instrument optique (40) en tant que position spatiale de l'instrument optique (40) dans un système de coordonnées du système OCT (100) sur la base du signal OCT (19) ;
la détermination (S300) d'une grandeur caractéristique de l'instrument optique (40) permettant l'identification de l'instrument optique sur la base de la position (42) de l'instrument optique (40) ; et
l'identification de l'instrument optique (40) parmi une pluralité d'instruments optiques précédemment connus présentant des grandeurs caractéristiques précédemment connues sur la base de la grandeur caractéristique déterminée.

2. Procédé d'une unité de commande (20) d'un système OCT (100) pour faire fonctionner le système OCT (100), le procédé comprenant les étapes de procédé suivantes :
l'acquisition (S100), au moyen du système OCT (100), d'un signal OCT (19) d'une structure anatomique introduite dans le chemin de faisceau (12) du système OCT (10) par un instrument optique (40) introduit dans le chemin de faisceau du système OCT (100) ;
la détermination (S200) d'une propriété de la structure anatomique sur la base du signal OCT (19) ;
la détermination (S300) d'une grandeur caractéristique de l'instrument optique (40) permettant l'identification de l'instrument optique sur la base d'une comparaison de la propriété déterminée de la structure anatomique avec une information de référence relative à la structure anatomique ; et
l'identification de l'instrument optique (40) parmi une pluralité d'instruments optiques précédemment connus présentant des grandeurs caractéristiques précédemment connues sur la base de la grandeur caractéristique déterminée.

3. Procédé selon la revendication 1, dans lequel une propriété géométrique de l'instrument optique (40) est en outre déterminée sur la base du signal OCT (19), et la grandeur caractéristique de l'instrument optique (40) est en outre déterminée sur la base de la propriété géométrique de l'instrument optique (40).

4. Procédé selon la revendication 3,
dans lequel des signaux OCT (19) sont acquis sur la base de signaux lumineux rétrodiffusés à partir d'une première surface (44) et/ou d'une seconde surface (45) de l'instrument optique (40),
dans lequel une forme de la première surface (44) et/ou une forme de la seconde surface (45) ainsi qu'une épaisseur (46) de l'instrument optique (40) sont déterminées sur la base des signaux OCT (19), et
dans lequel la grandeur caractéristique de l'instrument optique (40) est déterminée sur la base de la forme d'au moins l'une des surfaces (44, 45) et sur la base de l'épaisseur (46) de l'instrument optique (40).

5. Procédé selon la revendication 4, dans lequel une fonction de transfert optique de l'instrument optique (40) est déterminée sur la base de la forme d'au moins l'une des surfaces (44, 45) et sur la base de l'épaisseur (46) de l'instrument optique (40).

6. Procédé selon l'une quelconque des revendications 3 à 5,
dans lequel au moins un balayage linéaire destiné à l'acquisition d'une pluralité de signaux OCT (19) est effectué, et
dans lequel la propriété géométrique et/ou la position de l'instrument optique (40) sont déterminées sur la base de la pluralité de signaux OCT (19).

7. Procédé selon la revendication 6, dans lequel une pluralité de balayages linéaires sont effectués et dans lequel des directions de balayage de la pluralité de balayages linéaires sont respectivement tournées d'un angle plan les unes par rapport aux autres et/ou sont décalées dans la direction latérale.

8. Procédé selon la revendication 1, dans lequel une grandeur caractéristique de l'instrument optique (40) déterminée sur la base de la position (42) de l'instrument optique (40) comprend une extension d'un support (43) de l'instrument optique (40).

9. Procédé selon la revendication 1, dans lequel, sur la base du signal OCT (19), une propriété optique de l'instrument optique (40) est en outre déterminée, et la propriété optique (41) comprend au moins l'une parmi une propriété de diffusion, une propriété de dispersion, une réflectivité, une propriété de matériau et/ou un revêtement de l'instrument optique (44).

10. Procédé selon la revendication 1, comprenant en outre :
la sélection d'un instrument optique (40) parmi la pluralité d'instruments optiques précédemment connus au moyen d'une entrée utilisateur via une interface utilisateur (22) ;
l'émission d'un avis à l'intention de l'utilisateur lorsque la sélection basée sur l'entrée utilisateur est différente de l'instrument optique identifié (40) selon la revendication 1 ; et/ou
le remplacement de la sélection basée sur l'entrée utilisateur par l'instrument optique identifié (40) selon la revendication 1.

11. Procédé selon l'une quelconque des revendications précédentes, dans lequel le signal OCT (19) acquis et la grandeur caractéristique déterminée de l'instrument optique (40) introduit dans le chemin de faisceau (12) du système OCT (100), ou une grandeur caractéristique précédemment connue d'un instrument optique précédemment connu et sélectionnée sur la base de la grandeur caractéristique déterminée, sont utilisés pour la génération d'images et/ou pour l'évaluation quantitative du signal OCT (19).

12. Procédé selon l'une quelconque des revendications précédentes, dans lequel les étapes selon la revendication 1 s'effectuent de manière continue pendant l'acquisition d'un signal OCT (19) au moyen du système OCT (100), en réponse à la détection de l'introduction d'un instrument optique (40) dans le chemin de faisceau (12) du système OCT (100) ou en réponse à la détection d'une modification de l'échelle du signal OCT (19) acquis au cours d'une évolution temporelle du signal OCT (19).

13. Système OCT (100), comprenant :
une source lumineuse à large bande (11), de préférence un laser accordable ;
un interféromètre (14) conçu pour générer et superposer un faisceau échantillon (12) et un faisceau de référence (13) ;
un détecteur (18) conçu pour acquérir un motif d'interférence (17) généré par superposition d'un faisceau échantillon (12) et d'un faisceau de référence (13) sous la forme d'un signal OCT résolu dans le temps (19) ;
un mécanisme de balayage (16) conçu pour balayer le chemin de faisceau (12) du système OCT (100) ; et
une unité de commande (20) reliée à la source lumineuse (11) et au détecteur (18), l'unité de commande (20) étant conçue pour :
commander la source lumineuse (11) afin d'irradier un instrument optique (40) introduit dans un chemin de faisceau (12) du système OCT (100) ;
commander le détecteur (18) afin d'acquérir un signal OCT (19) de l'instrument optique (40) ;
déterminer une position de l'instrument optique (40) en tant que position spatiale de l'instrument optique (40) dans un système de coordonnées du système OCT (100) sur la base du signal OCT (19),
déterminer une grandeur caractéristique de l'instrument optique (40) qui permet l'identification de l'instrument optique sur la base de la position de l'instrument optique (40), et
identifier l'instrument optique (40) parmi une pluralité d'instruments optiques précédemment connus présentant des grandeurs caractéristiques précédemment connues sur la base de la grandeur caractéristique déterminée.

14. Système OCT (100), comprenant :
une source lumineuse à large bande (11), de préférence un laser accordable ;
un interféromètre (14) conçu pour générer et superposer un faisceau échantillon (12) et un faisceau de référence (13) ;
un détecteur (18) conçu pour acquérir un motif d'interférence (17) généré par superposition d'un faisceau échantillon (12) et d'un faisceau de référence (13) sous la forme d'un signal OCT résolu dans le temps (19) ;
un mécanisme de balayage (16) conçu pour balayer le chemin de faisceau (12) du système OCT (100) ; et
une unité de commande (20) reliée à la source lumineuse (11) et au détecteur (18), l'unité de commande (20) étant conçue pour :
commander la source lumineuse (11) afin d'irradier une structure anatomique introduite dans le chemin de faisceau (12) du système OCT (10) ;
commander le détecteur (18) afin d'acquérir un signal OCT (19) de la structure anatomique au moyen d'un instrument optique (40) introduit dans le chemin de faisceau du système OCT (100) ;
déterminer une propriété de la structure anatomique sur la base du signal OCT (19),
déterminer une grandeur caractéristique de l'instrument optique (40) qui permet l'identification de l'instrument optique sur la base d'une comparaison de la propriété déterminée de la structure anatomique avec une information de référence relative à la structure anatomique, et
identifier l'instrument optique (40) parmi une pluralité d'instruments optiques précédemment connus présentant des grandeurs caractéristiques précédemment connues sur la base de la grandeur caractéristique déterminée.

15. Système OCT (100) selon la revendication 13 ou 14, comprenant en outre :
une pluralité d'instruments optiques précédemment connus présentant des propriétés précédemment connues ;
une mémoire (21) contenant des informations relatives à une pluralité d'instruments optiques précédemment connus présentant des grandeurs caractéristiques précédemment connues ;
une interface utilisateur (22) conçue pour recevoir une entrée utilisateur ; et/ou
un moyen de sortie (23) conçu pour émettre un avertissement à l'intention de l'utilisateur.

16. Programme informatique comprenant des instructions qui, lors de leur exécution par une unité de commande (20) d'un système OCT (100) selon la revendication 13, amènent le système OCT (100) à mettre en œuvre un procédé selon la revendication 1 ou comprenant des instructions qui, lors de leur exécution par une unité de commande (20) d'un système OCT (100) selon la revendication 14, amènent le système OCT (100) à mettre en œuvre un procédé selon la revendication 2.
